Europäisches Patentamt

(19) European Patent Office

Office européen des brevets

(11) Publication number : **0 333 438 B1**

(12) **EUROPEAN PATENT SPECIFICATION**

(45) Date of publication of patent specification :
15.07.92 Bulletin 92/29

(51) Int. Cl.⁵ : **A61K 37/36, C12N 15/00**

(21) Application number : 89302509.8

(22) Date of filing : 14.03.89

(54) **Novel derivative of human growth hormone.**

(30) Priority : 15.03.88 US 168281

(43) Date of publication of application :
20.09.89 Bulletin 89/38

(45) Publication of the grant of the patent :
15.07.92 Bulletin 92/29

(84) Designated Contracting States :
AT BE CH DE ES FR GB GR IT LI NL SE

(56) References cited :
WO-A-87/01708
THE JOURNAL OF BIOLOGICAL CHEMISTRY,
vol. 256, no. 22, 25th November 1981, pages
11645-11650, US; U.J. LEWIS et al.:
"Alteredproteolytic cleavage of human
growth hormone as a result of deamidation"

(73) Proprietor : ELI LILLY AND COMPANY
Lilly Corporate Center
Indianapolis Indiana 46285 (US)

(72) Inventor : Becker, Gerald Wayne
5944 Crestview Avenue
Indianapolis Indiana 46220 (US)
Inventor : Riggin, Ralph Meridith
8810 Staghorn Road
Indianapolis Indiana 46260 (US)

(74) Representative : Hudson, Christopher Mark et
al
Erl Wood Manor
Windlesham Surrey GU20 6PH (GB)

## Description

This invention relates to a novel derivative of human growth hormone.

Large polyfunctional molecules such as proteins have the potential for forming a variety of derivatives of the original product. Indeed, modifications such as glycosylations and $\gamma$-carboxylations are well known and have been shown to have important physiological roles. Other modifications arise from the purification process. Degradation products also arise during storage of the protein. It is impossible a priori to know whether these structures will or will not have biological activity in their own right or the level and kind, if any, of such activity. Derivatives of pituitary derived human growth hormone (hGH) have been reported in the literature. Different size isomers of hGH have been detected in the plasma of both normal and acromegalic individuals. The predominant size isomer, the 22,000 dalton monomer, has been detected in several derivatized forms, including three proteolytically modified forms [Chramback, A., Yadley, R. A., Ben-David, M., and Rodbard, D. (1973) Endocrinology 93, 848-857; Singh, R. N. P., Seavey, B. K., Rice, V. P., Lindsey, T. T., and Lewis, U. J. (1974) Endocrinology 94, 883-891], an acetylated form and two deamidated forms [Lewis, U. J., Singh, R. N. P., Bonewald, L. F., Lewis, L. J., and Vanderlaan, W. P. (1979) Endocrinology 104, 1256-1265; Lewis, U. J., Singh, R. N. P., Bonewald, L. F., and Seavey, B. K. (1981) J. Biol. Chem. 256, 11645-11650]. Other hGH derivatives have been detected but have not been characterized. The recent use of recombinant DNA techniques to mass produce proteins of pharmaceutical interest has made it possible to attempt to prepare specifically modified products of the native recombinantly produced materials.

The availability of human growth hormone enabled the discovery of a novel desamido derivative of human growth hormone and development of a method for its production. It is to such derivative that this invention is directed. The compound of this invention is the desamido derivative of human growth hormone in which the asparagine (Asn) residue at position 149 is replaced by an aspartic acid (Asp) residue. For convenience, the compound of this invention is designated Asp[149]-human growth hormone, or Asp[149]-hGH. The compound of this invention unexpectedly exhibits a potency approximately equivalent to that of hGH itself and differs from the prior art in that the literature desamido derivatives of hGH have the asparagine residue in position 152 replaced by an aspartic acid residue or the glutamine (Gln) residue in position 137 replaced by a glutamic acid (Glu) residue [Lewis et al. (1981), supra].

As noted, this invention is directed to Asp[149]-hGH, and, as further noted, Lewis et al. (1981) J. Biol. Chem. 256, 11645-11650, describe two desamido derivatives of human growth hormone. The first replaces Asn[152] with ASP[152] and the second replaces Gln[137] with Glu[137]. No biological properties are reported for either molecules.

The compound of this invention in all measured respects is equipotent to the native hormone.

Asp[149]-hGH can be prepared from the native hormone by treatment with ammonium bicarbonate as described in the following:

Asp[149]-hGH was produced by dissolving 2 grams of biosynthetic human growth hormone (hGH) in 200 ml of 0.1M ammonium bicarbonate pH 9 and incubating at 37°C for 72 hours at which point the entire sample was lyophilized. The Asp[149]-hGH was purified by a two step procedure. The first step was an anion exchange column using Mono Q (1.6 X 10cm). The starting material was dissolved in 50 mM tris base at 16.7 mg/ml. After complete dissolution of the sample, acetonitrile was added to 10% of the final volume followed by 1-propanol to 30% of the final volume. This solution was purified in seven runs on the Mono Q® column with the protein load varying between 200 and 300 mg. Protein was eluted using a gradient generated from two solvents, A: 60% tris buffer (50 mM tris HCl pH 8), 10% acetonitrile; 30% 1-propanol and B: 60% tris buffer (50 mM tris HCl pH 8), 30% 1-propanol, 250 mM NaCl. The gradient was 0-25% B over 15 minutes, isocratic at 25% B for 10 minutes, and 25-50% B over 60 minutes. Pooled fractions were further purified by preparative reversed-phase HPLC using a column packed with Vydac®, $C_{18}$, 300 Å pore diameter silica and measuring 22.5 X 250 mm. The pools were adjusted to pH 7.5 and the 1-propanol concentration to 25% prior to loading onto the column. The amount of protein loaded varied between 140 and 240 mg. The protein was eluted from the column with a gradient generated from two solvents, A: 30% acetonitrile, 70% tris buffer (50 mM tris HCl pH 7.5) and B: 40% 1-propanol, 60% tris buffer (50 mM tris HCl pH 7.5). The gradient was 66% B to 72% B over 150 minutes. The temperature of the column was maintained at 45°C and the flow rate was 6 ml/min. Prior to loading the sample, the column was equilibrated with 66% B. Four columns were run as described and the fractions containing the Asp[149]-hGH were pooled, passed over a column packed with Sephadex® G-25 to remove the organic solvents and buffer salts, and lyophilized. The G-25 column was equilibrated and the protein eluted using ammonia-buffered water (pH 7.5).

The isolated compound was shown to be Asp[149]-hGH. Proof of this structure was accomplished by a peptide mapping procedure that has been described in the literature [Hsiung, H.M., Mayne N.G. and Becker G.W. (1986) Bio/Technology 4, 991-995.] Protein samples [1-2 mg/ml in tris acetate (50 mM, pH 7.5)] were digested with trypsin (TPCK-trypsin, Cooper Biomedical) at 37°C at an enzyme:substrate weight ratio of 1:25 for 16 hours. The resulting tryptic peptides were separated by reversed-phase HPLC on an Aquapore® RP-300 column (4.6 X 250 mm, Brownlee Labs) using a gradient generated from two solvents: A, 0.1% trifluoroacetic acid in water and B, 0.1% trifluoroacetic acid in acetonitrile. The gradient was 0-20% B in 20 minutes, 20-25% B in 20 minutes, and 25-50% in 25 minutes. The flow rate was 1.0 ml/min. A 100 ul aliquot of the tryptic digest was injected onto the column, and elution of the peptides was monitored spectrophotometrically at 214 nm.

Comparison of the peptides generated from ASP[149]-hGH with those arising from unmodified hGH revealed that all of the peptides were the same with one exception. This modified peptide was isolated from the trypsin digest of the derivative and shown by automated Edman degradation to have the sequence: Phe-Asp-Thr-Asp-Ser-His-Asn-Asp. This sequence matches amino acids 146 through 153 of hGH with the exception that, in unmodified hGH, the amino acid in position 149 is Asn instead of Asp. This establishes that the isolated compound is Asp[149]-hGH.

The biological activity of Asp[149]-hGH was assessed in hypophysectomized female Sprague-Dawley rats using the standard tibia assay [Marx. W., Simpson, M. E. and Evans, H. M. (1942) Endocrinology 30, 1-10; and Evans, H. M., Simpson, M. E., Marx, W. and Kirbrick, E. (1943) J. Endocrinology 32, 13-16]. Based on this analysis, the biological activity of ASP[149]-hGH is statistically indistinguishable from that of unmodified hGH, it shows anabotic activity.

## Claims

### Claims for the following Contracting States: AT, BE, CH, DE, FR, GB, IT, LI, NL, SE

1. Asp[149]-human growth hormone.

2. A pharmaceutical formulation comprising as an active ingredient Asp[149]-human growth hormone associated with one or more pharmaceutically-acceptable carriers or excipients therefor.

3. Asp[149]-human growth hormone for use in anabolic therapy.

### Claim for the following Contracting States: GR, ES

1. A process for preparing a pharmaceutical formulation which comprises admixing Asp[149]-human growth hormone with one or more pharmaceutical-acceptable carriers or excipients therefor.

## Patentansprüche

### Patentansprüche für folgenden Vertragsstaaren: AT, BE, CH, DE, FR, GB, IT, LI, NL, SE

1. Asp[149]-Humanwachstumshormon.

2. Pharmazeutische Formulierung aus einem Asp[149]-Wachstumshormon als Wirkstoff in Verbindung mit einem oder mehreren pharmazeutisch annehmbaren Trägern oder Hilfsstoffen hierfür.

3. Ssp[149]-Humanwachstumshormon zur Anwendung bei der anabolischen Therapie.

### Patentanspruch für folgenden Vertragsstaat: GR, ES

1. Verfahren zur Herstellung einer pharmazeutischen Formulierung durch Vermischen von Ssp[149]-Humanwachstumshormon mit einem oder mehreren pharmazeutisch annehmbaren Trägern oder Hilfsstoffen hierfür.

## Revendications

### Revendication pour les Etats contractants suivants: AT,BE,CH,DE,FR,GB,IT,LI,NL,SE

1. Hormone de croissance humaine-Asp[149].

2. Formulation pharmaceutique comprenant, comme ingrédient actif, l'hormone de croissance humaine-Asp[149] en association avec un ou plusieurs supports ou excipients pharmaceutiquement acceptables pour cette dernière.

3. Hormone de croissance humaine-Asp[149] pour être utilisée dans une thérapie anabolique.

### Revendication pour les Etats contractants suivants: GR ES

1. Procédé pour préparer une formulation pharmaceutique qui consiste à mélanger l'hormone de croissance humaine-Asp[149] avec un ou plusieurs supports ou excipients pharmaceutiquement acceptables pour cette dernière.